# EUROPEAN PATENT APPLICATION

(11) **EP 1 221 304 A2**
(43) Date of publication of application: **10.07.2002**
(21) Application number: 01129074.9
(22) Date of filing: 07.12.2001
(51) Int. Cl.: A61B 5/15, A61M 5/32

(54) **Blood collection assembly**

(30) Priority: 05.01.2001 US 260032 P; 13.11.2001 US 67107 P
(71) Applicant: Becton, Dickinson and Company, Franklin Lakes, New Jersey 07417 (US)
(72) Inventor: Crawford, Jamieson William Maclean, New York, New York (US); Livanos, Stefanie, Bethlehem, Pennsylvania 18017 (US)
(74) Representative: von Kreisler, Alek, Dipl.-Chem.

(57) **Abstract**

An automatically shieldable blood collection set is provided. The blood collection set includes a needle assembly having a hub to which a needle cannula is fixedly attached. A safety shield is telescoped relative to the hub and the needle cannula such that the hub and the needle cannula can be moved from a distal position where the needle cannula is exposed to a proximal position where the needle cannula is safely shielded. A spring is provided between the shield and the hub to propel the hub and needle cannula proximally relative to the shield and into surrounding relationship with the needle cannula. A retainer is provided for releasably holding the hub and needle cannula in a distal ready-to-use condition relative to the shield. An actuator releases the retainer and enables the hub and needle cannula to be propelled by the spring. A lock may be provided for preventing inadvertent re-exposure of the needle cannula.

## Description

### BACKGROUND OF THE INVENTION

### 1. Field of the Invention

The subject invention relates to a blood collection set having a needle cannula that can be retracted into a shield to safely shield the needle cannula.

### 2. Description of the Related Art

A prior art blood collection set includes a small diameter needle cannula having a pointed distal end and a proximal end mounted to a thermoplastic hub. Portions of the blood collection set near the hub may be provided with a pair of flexible wings. The wings can be folded into face-to-face engagement with one another to facilitate digital manipulation of the small needle cannula. The wings then can be folded away from one another and taped into face-to-face engagement with the skin of the patient near a puncture site. The prior art blood collection set further includes a flexible plastic tube that has one end connected to the hub and an opposed end connected to a fitting. The fitting can be placed in communication with a reservoir to which collected blood may be directed.

The needle cannula of the prior art blood collection set typically is shielded prior to and after use to prevent accidental sticks. Needle shields used with prior art blood collection sets have taken many forms. Typically, a prior art blood collection set is packaged with a rigid tubular cap telescoped over the needle cannula to prevent accidental sticks prior to use. This tubular cap is removed from the needle cannula immediately prior to use of the blood collection set. Most prior art blood collection sets further include a second shield that is telescoped over the needle cannula and hub. The second shield may include at least one slot through which wings of the prior art hub may extend. Thus, the medical technician who uses the prior art blood collection set will hold the wings of the needle hub in one hand and the shield in the other hand after removing the needle cannula from the patient or blood donor. The wings then are slid proximally relative to the shield, thereby drawing the needle cannula into the shield. Some prior art shields are configured to engage the wings when the needle cannula has been shielded to make a re-exposure of the needle cannula difficult.

The digital manipulation that is required to shield the used needle cannula of a prior art blood collection set creates the potential for generating the accidental needle stick that the shield is intended to avoid. In particular, it is undesirable to rely upon a shielding that requires two hands to be moved in opposite directions in proximity to the point of a used needle cannula. Accordingly, the inventors herein have recognized the desirability of providing an automatically shieldable needle cannula for a blood collection set.

### SUMMARY OF THE INVENTION

The subject invention relates to a blood collection set which comprises a needle cannula having a proximal end, a pointed distal end and a lumen extending therebetween. The blood collection set further includes a hub that may be molded from a thermoplastic material. The hub includes a proximal end, a distal end and a passage extending continuously therebetween. The distal end of the hub is securely mounted to the proximal end of the needle cannula. Thus the lumen through the needle cannula communicates with the passage through the hub.

The blood collection set may further include a length of flexible tubing having opposed proximal and distal ends. The distal end of the flexible tubing may be connected to the proximal end of the hub such that the lumen through the needle cannula and the passage through the hub both communicate with the passage through the flexible tubing. The flexible tubing further includes a proximal end that may be connected to a fitting. The fitting may comprise a needle cannula that enables the blood collection set to be placed in communication with a reservoir for receiving a sample of blood. The tubing and the fitting may be of conventional design.

The blood collection set may further include a substantially rigid generally tubular safety cap mounted over the needle cannula for protection against accidental needle sticks prior to use of the blood collection set. The safety cap may include a proximal end that is frictionally engaged with the hub. The rigid tubular safety cap may be removed immediately prior to use of the blood collection set.

The blood collection set further includes a safety shield that is telescoped over a portion of the hub. The safety shield and the hub are dimensioned to permit relative movement therebetween. More particularly, the combined hub and needle cannula can be moved from a distal position where the needle cannula is exposed for use and a proximal position where the needle cannula is within the safety shield. Biasing means are provided between the safety shield and the hub for urging the hub proximally relative to the safety shield. The biasing means may be a coil spring that surrounds a portion of the hub.

The blood collection set further comprises a retainer for releasably retaining the hub and needle cannula in the distal position relative to the safety shield and against the stored energy of the biasing means. Actuation of the retainer causes the biasing means to retract the hub and the needle cannula into the safety shield.

### DESCRIPTION OF THE DRAWINGS

FIG. 1 is a perspective view of a blood collection set in accordance with the subject invention.

FIG. 2 is a cross-sectional view taken along line 2-2 in FIG. 1.

FIG. 3 is a perspective view similar to FIG. 1, but showing the shield protectively locked around the needle cannula.

### DETAILED DESCRIPTION

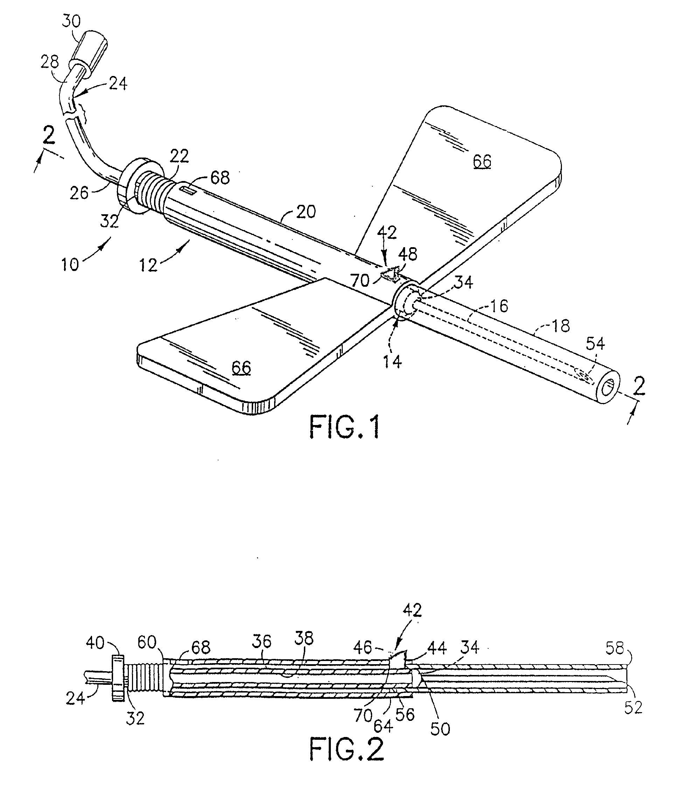

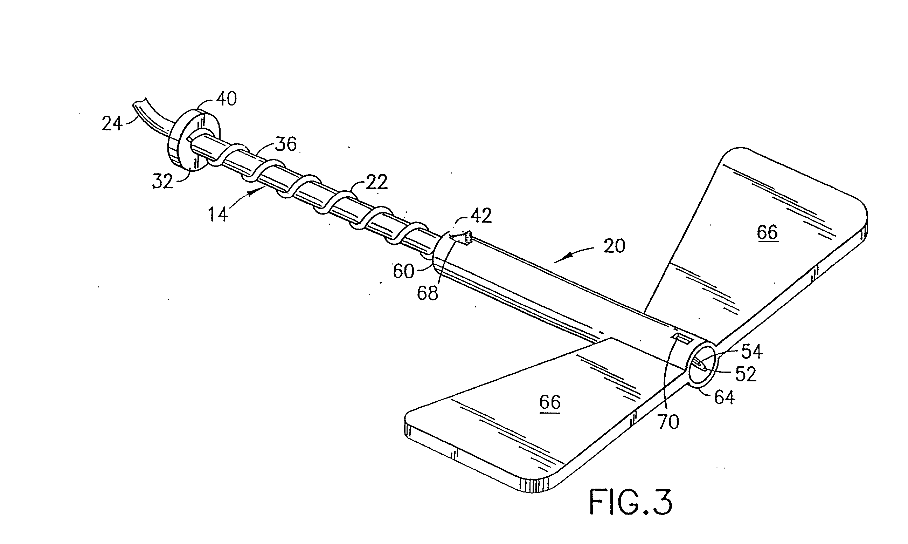

A blood collection set in accordance with the subject invention is identified generally by the numeral **10** in FIGS. 1-3. Blood collection set **10** includes a needle assembly **12** having a hub **14,** a needle cannula **16,** a safety cap **18,** a safety shield **20** and a spring **22.** Blood collection set **10** further includes a flexible tube 24 having a distal end **26** connected to needle assembly **12** and a proximal end **28** connected to a fitting **30.** Fitting **30** is configured to be placed in communication with a reservoir into which blood drawn by needle assembly **12** may be deposited. Flexible tube **24** and fitting **30** may be of conventional prior art construction.

Hub **14** is formed from a thermoplastic material and includes a proximal end **32,** a distal end **34** and a rigid tube **36** that extends axially between the ends. Tube **36** defines a passage **38** extending axially therethrough. An annular flange **40** is mounted on proximal end **32** of hub **14** and defines an outside diameter greater than the outside diameter of rigid tube **36** of hub **14.**

Hub **14** further includes an actuator **42** that projects radially outwardly from a location on hub **14** near distal end **34.** Actuator **42** defines a distal face **44** which, as explained further herein, functions as a retainer for releasably retaining shield **20** in a retracted position. Actuator **42** further includes a proximal face **46** which extends from outer surface **40** by a radial dimension that is less than the radial dimension of distal face **44.** A ramp face **48** extends angularly between distal face **44** and proximal face **46** of actuator **42.**

Needle cannula **16** includes a proximal end **50,** a pointed distal end **52** and a lumen **54** extending therebetween. Proximal end **50** is mounted securely in distal end **34** of hub **14,** such that lumen **54** communicates with passage **38** through hub **14** and with flexible tube **24.**

Safety cap **18** is a generally tubular structure that is unitarily molded from a rigid plastic material. Safety cap **18** has a proximal end **56** that is frictionally engaged at distal end **34** of hub **14** and a distal end **58** that extends distally beyond needle cannula **16** for preventing accidental sticks prior to use of blood collection set **10.**

Safety shield **20** is a substantially cylindrical tube having a proximal end **60** and a distal end **64.** A pair of flexible wings **66** project transversely from a location on safety shield **20** near distal end **64.** As shown in FIG. 1, wings **66** are substantially coplanar and enable needle assembly **12** to be taped to the skin of a patient in proximity to a puncture site. However, wings **66** can be folded approximately 90° toward one another to provide a convenient handle for gripping needle assembly **12.** Such gripping is helpful prior to use of needle assembly **12** when safety cap **18** is being removed and when needle cannula **16** is being inserted into a patient.

Safety shield **20** defines an inside diameter which is less than the outside diameter of flange **40** on hub **14.** However, the inside diameter of safety shield **20** is greater than the sum of the outside diameter of tube **36** of hub **14** plus the radial dimension of distal face **44** on actuator **42.** Thus, shield **20** can be slid over actuator **42** on hub **14.**

Safety shield **20** includes a proximal locking aperture **68** and a distal retaining aperture **70** each of which is dimensioned to receive actuator **42.** Safety shield **20** defines a length measured from locking aperture **68** to distal end **64** that exceeds the distance from actuator **42** to distal end **52** of needle cannula **16.**

Spring **22** surrounds rigid tube **36** of hub **14.** Spring **22** has an inside diameter smaller than the outside diameter of flange **40** and an outside diameter smaller than the inside diameter of safety shield **20.** Spring **22** is in a compressed state when hub **14** is in its distal position relative to safety shield 20, as shown in FIGS. 1 and 2.

Needle assembly **12** is used in a conventional manner by initially folding wings **66** toward one another to enable convenient digital manipulation of needle assembly **12.** Safety cap **18** then is slidably removed from needle cannula **16,** and needle cannula **16** is inserted into a patient. Wings **66** then may be folded flat against the skin of the patient and taped in place. Flexible tube **24** and fitting **30** will be used in conventional manner for connecting blood collection set **10** to a bag or other reservoir. Upon collection of a sufficient volume of blood, any tape that had held wings **66** to the skin of a patient will be removed, and needle assembly **12** then is withdrawn.

To prevent accidental sticks with the contaminated needle cannula **16,** the medical technician need merely squeeze actuator **42** and safety shield **20** between a thumb and forefinger. This squeezing will urge the actuator **42** within the safety shield **20,** and forces of spring **22** will urge hub **14** and needle cannula **16** proximally. Spring **22** will continue to propel hub **14** and needle cannula **16** in a proximal direction and into shielding relationship within safety shield **20.** After distal end **64** of safety shield **20** is distally beyond pointed distal end **52** of needle cannula **16,** actuator **42** will align with locking aperture **68** in shield **20.** The guiding of actuator **42** into locking aperture **68** can be achieved by a tapering of interior surface regions of safety shield **20** at locations opposite locking aperture **68.** In this condition, distal face **44** of actuator **42** will engage against distal portions of locking aperture **68** to prevent the re-exposure of needle cannula **16.** Additionally, distal portions of locking aperture **68** will engage against distal face **44** of actuator **42** to prevent a complete removal of safety shield **20** from hub **14.**

## Claims

1. A blood collection set comprising a fitting for connection to a blood collection reservoir, a flexible tube extending from said fitting and a needle assembly connected to an end of the flexible tube remote from the fitting, said needle assembly comprising:
a hub having a proximal end connected to said flexible tube, a distal end and a passage extending therebetween for providing fluid communication with said flexible tube;
a needle cannula having a proximal end rigidly connected to said distal end of said hub, a pointed distal end and a lumen extending therebetween, said lumen providing fluid communication with said passage through said hub;
a shield telescoped with said hub and said needle cannula, such that said hub and said needle cannula are movable from a distal position where said needle cannula is exposed to a proximal position where said needle cannula is shielded;
a spring captured between portions of said hub and said shield and operative for propelling said hub and said needle cannula into said proximal position relative to said shield;
a retainer for releasably retaining the hub and said needle cannula in the distal position; and
an actuator for selectively releasing said retainer.

2. The blood collection set of Claim 1, further comprising a lock for locking said hub and said needle cannula in said proximal position.

3. The blood collection set of Claim 1, wherein said shield is substantially tubular, and wherein said blood collection set further comprises a pair of flexible wings projecting transversely from said shield.

4. The blood collection set of Claim 3, wherein the shield includes opposed proximal and distal ends, the wings projecting transversely from the shield at a location substantially adjacent the distal end of the shield.

5. The blood collection set of Claim 1, wherein the shield is substantially tubular with opposed proximal and distal ends, a distal aperture extending through the shield at a location in proximity to the distal end, a projection extending outwardly from the needle hub and passing into the distal aperture adjacent the shield when the hub and the needle cannula are in the distal position, the retainer defining a first surface on the projection disposed for engaging the shield adjacent the distal aperture in the shield, the actuator defining a second surface on the projection extending outwardly from the shield and being depressible sufficiently for disengaging said retainer from said portions of said shield adjacent said aperture.

6. The blood collection set of Claim 5, wherein the projection further comprises a ramp face extending from the actuator surface on the projection to the retainer surface on the projection, the ramp face being aligned at an acute angle to the needle cannula.

7. The blood collection set of Claim 5, wherein the shield further comprises a proximal aperture extending therethrough at a location substantially adjacent the proximal end of the shield, the projection being dimensioned for locked engagement in the proximal aperture for preventing distal movement of the hub and the needle cannula relative to the shield.

8. A needle assembly comprising:
a hub having a proximal end, distal end and a passage extending therebetween for providing fluid communication through said hub;
a needle cannula mounted to the distal end of the hub;
a shield telescoped with said hub and said needle cannula, such that said hub and said needle cannula are movable from a distal position where said needle cannula is exposed to a proximal position where said needle cannula is enclosed within said shield;
a spring captured between portions of said hub and said shield and being operative for propelling said hub and said needle cannula into said proximal position relative to said shield;
a retainer for releasably retaining the hub and said needle cannula in the distal position; and
an actuator for selectively releasing said retainer.

9. The needle assembly of Claim 8, further comprising a lock for locking said hub and said needle cannula in said proximal position.

10. The needle assembly of Claim 8, wherein said shield is substantially tubular, and wherein said blood collection set further comprises a pair of flexible wings projecting transversely from said shield.

11. The needle assembly of Claim 10, wherein the shield includes opposed proximal and distal ends, the wings projecting transversely from the shield at a location substantially adjacent the distal end of the shield.

12. The needle assembly of Claim 8, wherein the shield is substantially tubular with opposed proximal and distal ends, a distal aperture extending through the shield in proximity to the distal end, a projection extending outwardly from the needle hub and passing into the distal aperture of the shield when the hub and the needle cannula are in the distal position, the retainer defining a first surface on the projection disposed for engaging the shield adjacent the distal aperture in the shield, the actuator defining a second surface on the projection extending outwardly from the shield and being depressible sufficiently for disengaging said retainer from said portions of said shield adjacent said aperture.

13. The needle assembly of Claim 12, wherein the projection further comprises a ramp face extending from the actuator surface on the projection to the retainer surface on the projection, the ramp face being aligned at an acute angle to the needle cannula.

14. The needle assembly of Claim 12, wherein the shield further comprises a proximal aperture extending therethrough at a location substantially adjacent the proximal end of the shield, the projection being dimensioned for locked engagement in the proximal aperture for preventing distal movement of the hub and the needle cannula relative to the shield.
